(19) Europäisches Patentamt
European Patent Office
Office européen des brevets

(11) Veröffentlichungsnummer: **0 036 544**
**B1**

(12) # EUROPÄISCHE PATENTSCHRIFT

(45) Veröffentlichungstag der Patentschrift: **25.01.84**

(21) Anmeldenummer: **81101699.7**

(22) Anmeldetag: **09.03.81**

(51) Int. Cl.³: **C 07 D 231/12,** C 07 D 233/61, C 07 D 249/04, C 07 D 249/08, C 07 D 257/04, C 07 D 295/12, C 07 D 405/06, C 07 D 409/06, A 01 N 43/34, A 01 N 43/48, A 01 N 43/64

(54) **Verfahren zur Herstellung von substituierten N-Azolylmethyl-halogen-acetaniliden.**

(30) Priorität: **22.03.80 DE 3011084**

(43) Veröffentlichungstag der Anmeldung: **30.09.81 Patentblatt 81/39**

(45) Bekanntmachung des Hinweises auf die Patenterteilung: **25.01.84 Patentblatt 84/4**

(84) Benannte Vertragsstaaten: **BE CH DE FR GB IT LI NL**

(56) Entgegenhaltungen:
**EP - A - 0 008 057**
**EP - A - 0 008 091**
**DE - B - 1 542 950**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Schmidt, Thomas, Dr., Am Bandenfeld 72, D-5657 Haan 1 (DE)**
Erfinder: **Thomas, Rudolf, Dr., Dellbusch 269, D-5600 Wuppertal 2 (DE)**
Erfinder: **Schulze, Andreas, Dr., Voiswinkeler Strasse 35, D-5060 Bergisch-Gladbach 2 (DE)**

**EP 0 036 544 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von substituierten N-Azolylmethylhalogenacetaniliden

Die vorliegende Erfindung betrifft ein neues, vorteilhaftes Verfahren zur Herstellung von bekannten substituierten N-Azolylmethylhalogenacetaniliden mit herbizider Wirksamkeit.

Es ist bereits bekannt geworden, dass man bestimmte N-Azolylmethylhalogenacetanilide erhalten kann, wenn man Aniline mit Paraformaldehyd in Gegenwart katalytischer Mengen Kaliumhydroxid umsetzt, die entstehenden Phenylazomethine mit einem Halogenacethalogenid versetzt (vgl. US-Patentschriften Nrn. 3630716 und 3637847) und die entstehenden N-Halogenmethyl-α-halogenacetanilide mit entsprechenden Heterocyclen gegebenenfalls in Gegenwart eines Säurebindemittels und in Gegenwart eines inerten organischen Lösungsmittels bei Temperaturen zwischen 0 und 200°C gemäss folgendem Reaktionsschema a umsetzt (vgl. US-PS Nrn. 3630716 und 3637847, DE-OS Nrn. 2648008 und 2704281):

*Reaktionsschema (a)*

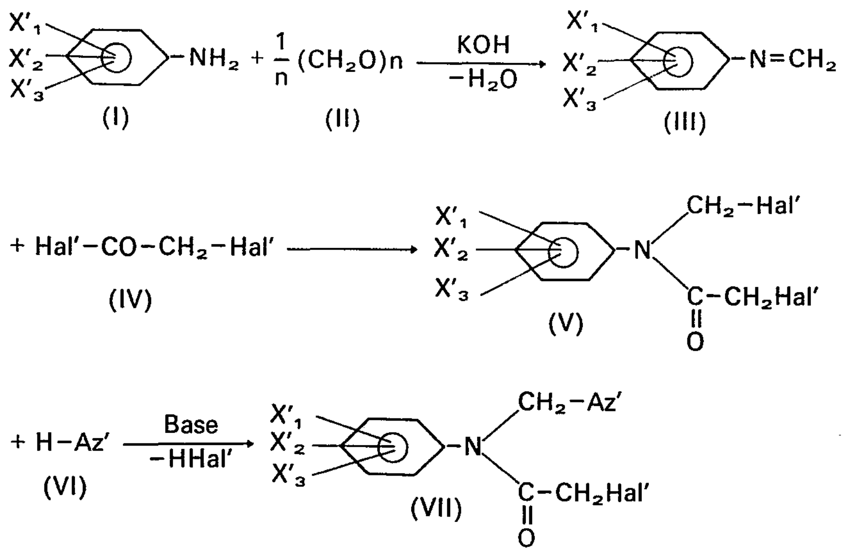


Az' = über ein Ringstickstoffatom gebundener N– haltiger heterocyclischer Ring;

$X'_1$, $X'_2$, $X'_3$ = Wasserstoff, Alkyl, Alkoxy, Halogenalkyl, Alkoxyalkyl, u.a., wobei $X'_1$, $X'_2$ und $X'_3$ gleichartig oder verschieden sind;

Hal' = Halogen.

Dieses Verfahren wurde nur zur Synthese von solchen Acetaniliden angewandt, in denen der Azolylrest über eine Methylengruppe mit dem verbleibenden Molekülteil verbunden ist. Gegen die analoge Herstellung von Verbindungen mit einer:

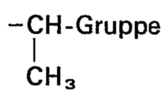

zwischen dem Azolylrest und dem Stickstoffatom der Anilideinheit sprach die Tatsache, dass sich die in der ersten Stufe der Umsetzung entstehenden Ethylidenamine bekanntermassen durch eine grosse Reaktionsfähigkeit auszeichnen. Es war nämlich anzunehmen, dass der als Ausgangsstoff benötigte Acetaldehyd unter den Umsetzungsbedingungen nicht das gewünschte Ethylidenamin liefert, sondern Kondensationsreaktionen eingeht.

Ferner geht aus der DE-B Nr. 1542950 hervor, dass sich Schiffbasen der Formel III, in denen beide ortho-Positionen des Phenylringes substituiert sind und mindestens einer der beiden Substituenten für tert.-Butyl steht, mit Halogenacetylhalogeniden zu Halogenacetaniliden entsprechend den Stoffen der Formel V umsetzen lassen. Über die Herstellung der Ausgangsmaterialien sind jedoch keine Angaben vorhanden. Ausserdem wird nicht die Synthese von solchen Stoffen beschrieben, in denen der Anilidstickstoff eine:

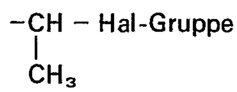

trägt.

Weiterhin ist bereits die Herstellung solcher Verbindungen der Formel VII bekannt geworden, in denen die Methylengruppe des Azolylmethylrestes substituiert ist (vgl. DE-A Nrn. 2835156 und 2835157 bzw. EP-A Nrn. 8091 und 8057). So lassen sich derartige substituierte N-Pyrazolylmethylhalogenacetanilide der Formel X erhalten, wenn man Halogenacetanilide der Formel VIII mit entsprechenden Pyrazolderivaten in Gegenwart eines Säurebinders, wie beispielsweise eines Alkalihydroxids, und gegebenenfalls in Gegenwart eines inerten organischen Lösungsmittels bei Temperaturen zwischen −70 und +100°C nach folgendem Reaktionsschema b umsetzt:

*Reaktionsschema (b)*

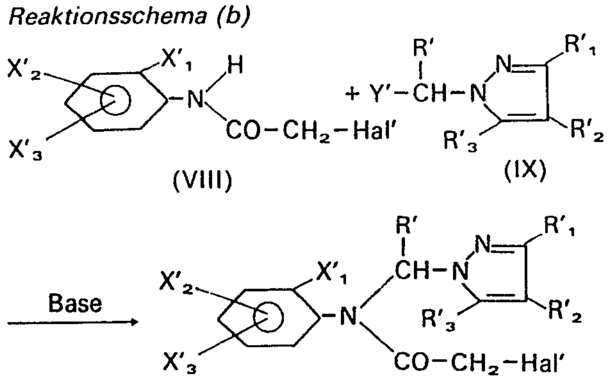


(VIII) (IX) (X)

R' = Alkyl, Cycloalkyl, Halogenalkyl, Alkoxyalkyl, Alkenyl, Alkinyl, gegebenenfalls substituiertes Phenyl;

$R'_1$, $R'_2$, $R'_3$ = Wasserstoff, Alkyl, Halogen, Alkoxy, wobei $R'_1$, $R'_2$ und $R'_3$ gleichartig oder verschieden sind;

Y' = Halogen, Mesylat- oder Tosylat-Rest;

$X'_1$, $X'_2$, $X'_3$ und Hal' = Bedeutung wie oben angegeben.

Dieses Verfahren gemäss Reaktionsschema b hat jedoch den Nachteil, dass es insgesamt 4 Stufen umfasst. So ist zunächst die Umsetzung von Azol mit Aldehyd erforderlich, dann die Überführung des entstehenden Carbinols in eine Verbindung der Formel IX, ausserdem die Reaktion von Anilinderivat mit Halogenacetylhalogenid und schliesslich die Umsetzung der Komponenten der Formeln VIII und IX. Nachteilig ist auch, dass die als Vorprodukte benötigten Carbinole nicht gut zugänglich sind, denn es lassen sich relativ wenige Azole mit Aldehyden zu Carbinolen umsetzen und anschliessend in Verbindungen der Formel IX überführen. Darüber hinaus sind auch die Ausbeuten bei dem Verfahren gemäss Reaktionsschema b nicht in allen Fällen befriedigend.

Die substituierten N-Pyrazolylmethylhalogenacetanilide der Formel X können auch hergestellt werden, indem man Aniline der Formel I zunächst mit Pyrazolylderivaten der Formel IX in Gegenwart eines Säurebinders, wie insbesondere einem Überschuss an Anilin, umsetzt und anschliessend die erhaltenen Aniline der Formel XI mit einem Halogenacethalogenid der Formel IV in Gegenwart eines inerten organischen Lösungsmittels, wie beispielsweise Toluol oder Dimethylformamid, gegebenenfalls in Gegenwart eines Säurebindemittels, wie z.B. Kaliumcarbonat, bei Temperaturen zwischen 20 und 100°C gemäss folgendem Reaktionsschema c umsetzt:

*Reaktionsschema (c)*

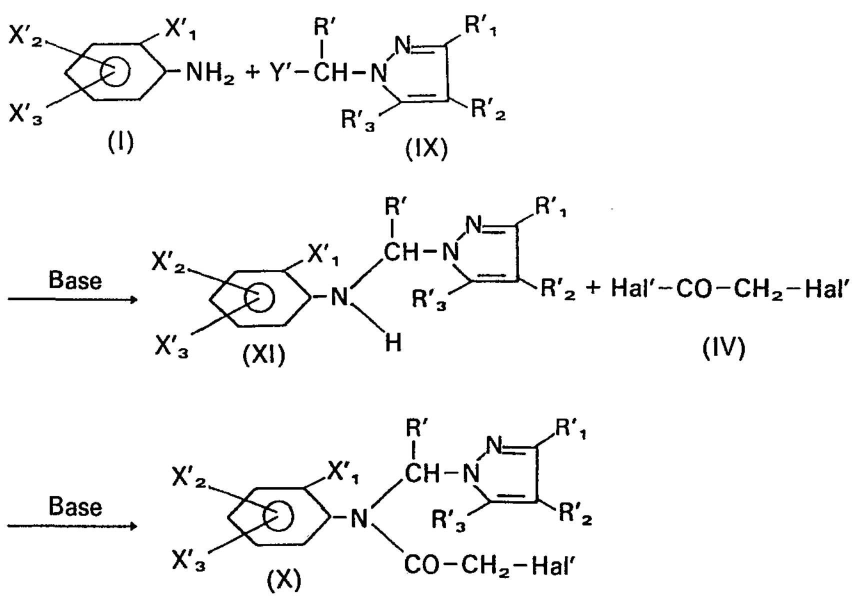


(I) (IX) (XI) (IV) (X)

$X'_1$, $X'_2$, $X'_3$, Y', R', $R'_1$, $R'_2$, $R'_3$ und Hal' = Bedeutung wie oben angegeben.

Dieses Verfahren gemäss Reaktionsschema c hat jedoch den Nachteil, dass es nicht immer in befriedigenden Ausbeuten abläuft. Das Halogenacetanilid der Formel IV hat die Möglichkeit zu einer unerwünschten Nebenreaktion, indem es nicht am Anilinstickstoff angreift, sondern das freie Stickstoffatom des Pyrazols acyliert.

Es wurde nun gefunden, dass man substituierte N-Azolylmethylhalogenacetanilide der Formel:

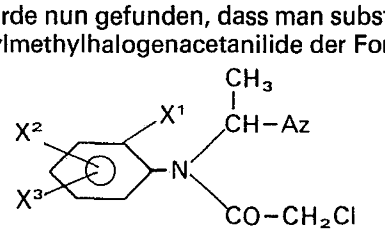
(XII)

in welcher

Az für einen über ein Stickstoffatom gebundenen, gegebenenfalls durch Chlor, Alkyl mit 1 bis 4 Kohlenstoffatomen und/oder Alkoxy mit 1 bis 4 Kohlenstoffatomen substituierten Pyrazol-1-yl oder 1,2,4-Triazol-1-yl-Rest steht, und

$X^1$, $X^2$ und $X^3$ gleich oder verschieden sind und für Wasserstoff, Alkyl mit 1 bis 4 Kohlenstoffatomen oder für Chlor stehen,

dadurch erhält, dass man in einer 1. Stufe Aniline der Formel:

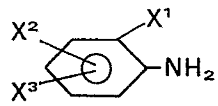

(XIII)

in welcher

$X^1$, $X^2$ und $X^3$ die oben angegebene Bedeutung haben,

mit Acetaldehyd der Formel:

$$H_3C-CH=O \qquad (XIV)$$

gegebenenfalls in Gegenwart eines Verdünnungsmittels und gegebenenfalls in Gegenwart von Dimethylbenzylamin als Katalysator bei Temperaturen zwischen −10 und +120°C umsetzt, dann in einer 2. Stufe die entstehenden Azomethine der Formel:

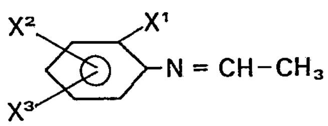

(XV)

in welcher

$X^1$, $X^2$ und $X^3$ die oben angegebene Bedeutung haben,

mit Chloracetylchlorid der Formel:

$$Cl-CO-CH_2Cl \qquad (XVI)$$

gegebenenfalls in Gegenwart eines Verdünnungsmittels bei Temperaturen zwischen −20 und +50°C umsetzt, und in einer 3. Stufe die entstehenden substituierten N-Halogenmethylhalogenacetanilide der Formel:

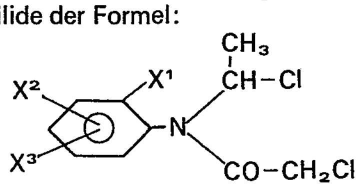

(XVII)

in welcher

$X^1$, $X^2$ und $X^3$ die oben angegebene Bedeutung haben,

ohne vorherige Isolierung direkt mit Azolen der Formel:

$$H\ Az \qquad (XVIII)$$

in welcher

Az die oben angegebene Bedeutung hat,

gegebenenfalls in Gegenwart eines Verdünnungsmittels und in Gegenwart eines Säurebindemittels bei Temperaturen zwischen −10 und +80°C umsetzt, wobei die Azole der Formel XVIII auch in Form ihrer Alkalimetallsalze eingesetzt werden können.

Es ist als ausgesprochen überraschend zu bezeichnen, dass das erfindungsgemässe Verfahren in guten Ausbeuten verläuft. Aufgrund der hohen Methylenaktivität des Acetaldehyds der Formel XIV wäre in der 1. Stufe der erfindungsgemässen Umsetzung eine Aldolkondensation zu höher kondensierten Derivaten möglich gewesen (vgl. hierzu auch „Chem. Rev." *26*, 297-333 [1940]).

Ausserdem wäre in der 3. Stufe der erfindungsgemässen Umsetzung eine Dehydrohalogenierungsreaktion an den substituierten N-Halogenmethylhalogenacetaniliden der Formel XVII zu erwarten gewesen.

Das erfindungsgemässe Verfahren weist eine Reihe von Vorteilen auf. Es handelt sich um eine einfach durchführbare Dreistufenreaktion, bei der die beiden letzten Stufen ohne vorherige Isolierung des jeweiligen Zwischenproduktes der Formel XVII vorgenommen werden. Im Zuge der Umsetzung kommt es kaum zur Bildung von Nebenprodukten, und die Ausbeuten an Verbindungen der Formel XII sind allgemein gut.

Die nach dem erfindungsgemässen Verfahren herstellbaren substituierten N-Azolylmethylhalogenacetanilide der Formel XII sind bekannt (vgl. DE-A Nrn. 2835156 und 2835157 bzw. EP-A Nrn. 8091 und 8057).

Verwendet man beispielsweise 2,6-Dimethylanilin und Acetaldehyd als Ausgangsstoffe und Chloracetylchlorid sowie Pyrazol als Reaktionskomponenten, so kann der Ablauf des erfindungsgemässen Verfahrens durch das folgende Formelschema wiedergegeben werden:

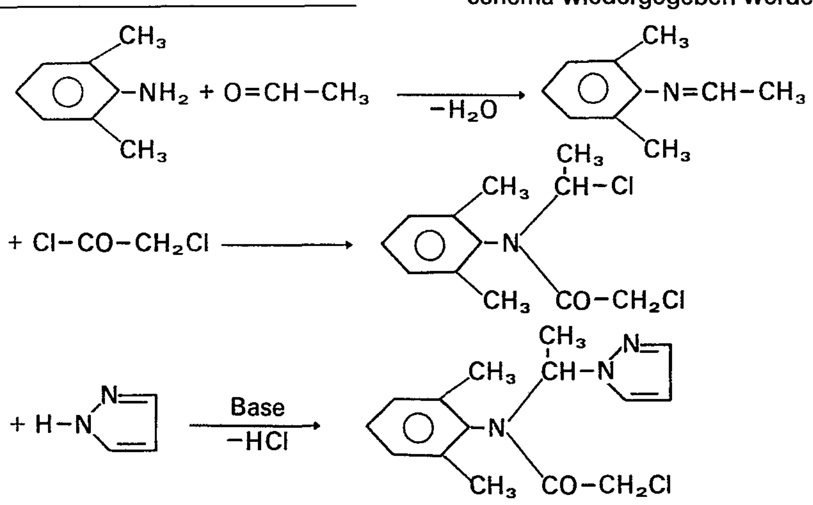

Die bei der Durchführung der 1. Stufe des erfindungsgemässen Verfahrens als Ausgangsstoffe zu verwendenden Aniline sind durch die Formel XIII allgemein definiert. In dieser Formel stehen $X^1$, $X^2$ und $X^3$ für diejenigen Reste, die bereits im Zusammenhang mit der Beschreibung der erfindungsgemäss herstellbaren Stoffe der Formel (XII) für diese Reste genannt wurden.

Die Aniline der Formel XIII sind allgemein bekannte Verbindungen der organischen Chemie. Als Beispiele seien genannt:

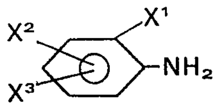 (XIII)

| $X^1$ | $X^2$ | $X^3$ |
|---|---|---|
| H | H | H |
| $CH_3$ | 6-$CH_3$ | H |
| $CH_3$ | 6-$C_2H_5$ | H |
| $C_2H_5$ | 6-$C_2H_5$ | H |
| i-$C_3H_7$ | H | H |
| t-$C_4H_9$ | H | H |
| $C_2H_5$ | H | H |
| $CH_3$ | H | H |
| $CH_3$ | 3-$CH_3$ | H |
| $CH_3$ | 5-$CH_3$ | H |
| $CH_3$ | 4-$CH_3$ | 6-$CH_3$ |
| $C_2H_5$ | 4-$CH_3$ | 6-$CH_3$ |
| sek.-$C_4H_9$ | H | H |
| H | H | H |
| H | 3-$CH_3$ | 5-$CH_3$ |
| $CH_3$ | 6-Cl | H |
| t-$C_4H_9$ | 6-Cl | H |
| H | 3-Cl | 4-Cl |
| Cl | 6-Cl | H |

Die bei der Durchführung der 2. Stufe der erfindungsgemässen Umsetzung entstehenden substituierten N-Halogenmethylhalogenacetanilide der Formel XVII stellen allgemein interessante Zwischenprodukte dar. Als Beispiele seien genannt:

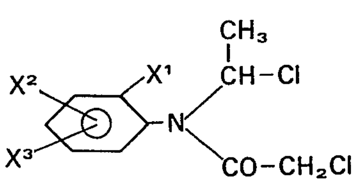 (XVII)

| $X^1$ | $X^2$ | $X^3$ |
|---|---|---|
| $CH_3$ | 6-$CH_3$ | H |
| $C_2H_5$ | 6-$CH_3$ | H |
| $C_2H_5$ | 6-$C_2H_5$ | H |
| $CH_3$ | H | H |
| $CH_3$ | 3-$CH_3$ | 4-$CH_3$ |
| $CH_3$ | 3-$CH_3$ | H |
| $CH_3$ | 5-$CH_3$ | H |
| $CH_3$ | 6-Cl | H |
| H | 3-Cl | 4-Cl |

Die ausserdem für die 3. Stufe der erfindungsgemässen Umsetzung als Reaktionskomponenten zu verwendenden Azole sind durch die Formel XVIII allgemein definiert. In dieser Formel steht Az für diejenigen Reste, die bereits im Zusammenhang mit der Beschreibung der erfindungsgemäss herstellbaren Stoffe der Formel XII für diesen Substituenten genannt wurden.

Die Azole der Formel XVIII sind allgemein bekannte Verbindungen der organischen Chemie. Als Beispiele seien genannt:

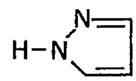, 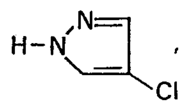, 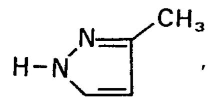,

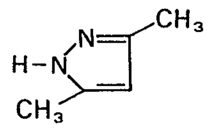, 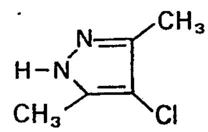,

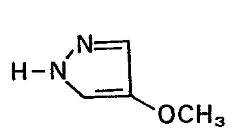, 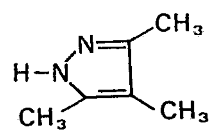,

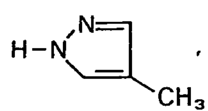, 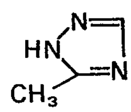, 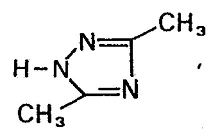,

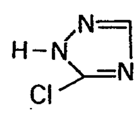, 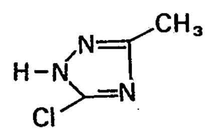,

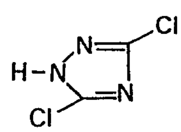, 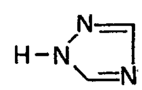,

Als Verdünnungsmittel kommen für die 1. Stufe des erfindungsgemässen Verfahrens alle inerten Lösungsmittel in Frage. Hierzu gehören vorzugsweise Alkohole, wie Methanol, Ethanol und tert.-Butanol; Ether, wie insbesondere Tetrahydrofuran und Dioxan; halogenierte Kohlenwasserstoffe, wie Methylenchlorid, sowie aromatische Kohlenwasserstoffe, wie Benzol, Toluol oder Xylol.

Die Reaktionstemperaturen können bei der Durchführung der 1. Stufe des erfindungsgemässen Verfahrens in einem grösseren Bereich variiert werden. Man arbeitet bei Temperaturen zwischen −10 und +120°C.

Bei der Durchführung der 1. Stufe des erfindungsgemässen Verfahrens setzt man vorzugsweise auf 1 mol Anilin der Formel XIII 1 bis 2 mol Acetaldehyd der Formel XIV und gegebenenfalls 0,01 bis 0,1 mol Katalysator ein. Das entstehende Reaktionswasser wird in üblicher Art und Weise entfernt, wie z.B. durch Zugabe wasserentziehender Mittel, beispielsweise Natriumsulfat oder Magnesiumsulfat oder durch physikalische Methoden, wie z.B. Erhitzen am Wasserabscheider, azeotrope Destillation gegebenenfalls im Vakuum oder auch Ausfrieren. Die Isolierung der Azomethine der Formel XV erfolgt in üblicher Weise.

Als Verdünnungsmittel kommen für die 2. Stufe des erfindungsgemässen Verfahrens inerte organische Lösungsmittel in Frage. Hierzu gehören vorzugsweise Ketone, wie insbesondere Aceton und Methylethylketon; Nitrile, wie insbesondere Acetonitril; Ether, wie Tetrahydrofuran oder Dioxan; Ester, wie insbesondere Essigester; halogenierte Kohlenwasserstoffe, wie Methylenchlorid, sowie aromatische Kohlenwasserstoffe, wie Benzol und Toluol.

Die Reaktionstemperaturen können bei der Durchführung der 2. Stufe des erfindungsgemässen Verfahrens in einem grösseren Bereich variiert werden. Man arbeitet bei Temperaturen zwischen −20 und +50°C.

Bei der Durchführung der 2. Stufe des erfindungsgemässen Verfahrens setzt man vorzugsweise auf 1 mol Azomethin der Formel XV 1 bis 1,5 mol Chloracetylchlorid der Formel XVI ein. Die entstehenden substituierten N-Halogenmethylhalogenacetanilide werden ohne Isolierung direkt weiter umgesetzt.

Als Verdünnungsmittel kommen für die 3. Stufe des erfindungsgemässen Verfahrens inerte organische Lösungsmittel in Frage. Hierzu gehören vorzugsweise die bei der 2. Stufe bereits vorzugsweise genannten Solvenzien.

Als Säurebindemittel können alle üblicherweise verwendbaren anorganischen und organischen Säureakzeptoren eingesetzt werden. Hierzu gehören vorzugsweise Alkalicarbonate, wie Natrium- und Kaliumcarbonat oder Natriumhydrogencarbonat; ferner niedere tertiäre Alkylamine, Aralkylamine, aromatische Amine oder Cycloalkylamine, wie Triethylamin, Dimethylbenzylamin und Pyridin. Es ist aber auch möglich, einen entsprechenden Überschuss an Azol zu verwenden, bzw. das Azol in Form eines Alkalimetallsalzes einzusetzen.

Die Reaktionstemperaturen können bei der Durchführung der 3. Stufe des erfindungsgemässen Verfahrens in einem grösseren Bereich variiert werden. Man arbeitet bei Temperaturen zwischen −10 und +80°C.

Bei der Durchführung der 3. Stufe des erfindungsgemässen Verfahrens setzt man auf 1 mol substituiertes N-Halogenmethylhalogenacetanilid der Formel XVII 1 bis 2,5 mol Azol der Formel XVIII und 1 mol Säurebinder ein. Die Isolierung der Endprodukte erfolgt in allgemein üblicher Art und Weise.

Das erfindungsgemässe Verfahren lässt sich auch als Eintopfreaktion durchführen.

Die erfindungsgemäss herstellbaren Wirkstoffe der Formel XII zeichnen sich durch sehr gute herbizide, insbesondere selektiv-herbizide Wirksamkeit aus, die der des bekannten 2,6-Diethyl-N-methoxymethylchloracetanilid (vgl. R. Wegler, „Chemie der Pflanzenschutz- und Schädlingsbekämpfungsmittel", Bd. 5, S. 255, Springer-Verlag [1977]) überlegen ist (vgl. das folgende Beispiel A).

Die erfindungsgemäss herstellbaren Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemässen Stoffe als totale oder selektive Herbizide wirken, hängt im wesentlichen von der angewendeten Menge ab.

Die erfindungsgemässen Wirkstoffe können z.B. bei den folgenden Pflanzen verwendet werden:

Dikotyle Unkräuter der Gattungen: *Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.*

Dikotyle Kulturen der Gattungen: *Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.*

Monokotyle Unkräuter der Gattungen: *Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.*

Monokotyle Kulturen der Gattungen: *Oryza, Zea, Triticum, Hordeum, Avena, Secale, Sorghum, Panicum, Saccharum, Ananas, Asparagus, Allium.*

Die Verwendung der erfindungsgemäss herstellbaren Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions/Emulsions-Konzentrate, wirkstoffimprägnierte Natur- und synthetische Stoffe, Feinverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden

Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde, und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine, wie Calcit, Marmor, Bims, Sepiolith, Dolomit sowie synthetische Granulate aus anorganischen und organischen Mehlen sowie Granulate aus organischem Material, wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyethylenfettsäureester, Polyoxyethylenfettalkoholether, z.B. Alkylarylpolyglykolether, Alkylsulfonate, Alkylsulfate, Arylsulfonate sowie Eiweisshydrolysate; als Dispergiermittel kommen in Frage: z.B. Ligninsulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabikum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90%.

Die erfindungsgemäss herstellbaren Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfrass, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Giessen, Spritzen, Sprühen, Streuen.

Die erfindungsgemäss herstellbaren Wirkstoffe können sowohl vor als auch nach dem Auflaufen der Pflanzen appliziert werden. Die Anwendung wird vorzugsweise vor dem Auflaufen der Pflanzen, also im *pre-emergence*-Verfahren, vorgenommen. Sie können auch vor der Saat in den Boden eingearbeitet werden.

Die angewandte Wirkstoffmenge kann in grösseren Bereichen schwanken. Sie hängt im wesentlichen von der Art des gewünschten Effekts ab. Im allgemeinen liegen die Aufwandmengen zwischen 0,05 und 10 kg Wirkstoff/ha, vorzugsweise zwischen 0,1 und 5 kg/ha.

Wirkung und Einsatzmöglichkeiten der erfindungsgemäss herstellbaren Stoffe zur Unkrautbekämpfung gehen aus dem Beispiel A hervor.

Das erfindungsgemässe Verfahren sei anhand der folgenden Herstellungsbeispiele erläutert.

*Herstellungsbeispiele*

*Beispiel 1*

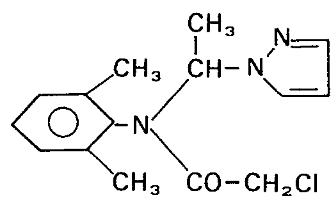

(XII-1)

*(Stufe 1)*

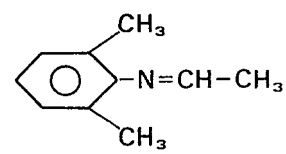

(XV-1)

121 g (1 mol) 2,6-Dimethylanilin in 200 ml Methylenchlorid werden mit 480 g (3 mol) Natriumsulfat und 3 ml Dimethylbenzylamin versetzt. Dazu werden bei 0°C 88 g (2 mol) Acetaldehyd zugegeben. Man lässt 4 h bei 0°C rühren, filtriert und engt das Filtrat im Vakuum bei maximal 20°C Badtemperatur ein. Man erhält 140 g (95,2% der Theorie) N-Ethyliden-2,6-dimethylanilin als rötliches Öl.

*(Stufe 2)*

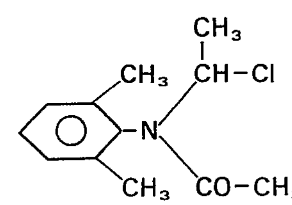

(XVII-1)

Zu einer Lösung von 124,2 g (1,1 mol) Chloracetylchlorid in 100 ml Toluol werden (unter die Oberfläche) bei Temperaturen zwischen −10 und +10°C 147 g (1 mol) N-Ethyliden-2,6-dimethylanilin zugetropft. Man lässt 3 h bei 0°C bis Raumtemperatur nachrühren. Das entstehende 2,6-Dimethyl-N-(1-chloreth-1-yl)chloracetanilid wird ohne Isolierung direkt weiter umgesetzt.

*(Stufe 3)*

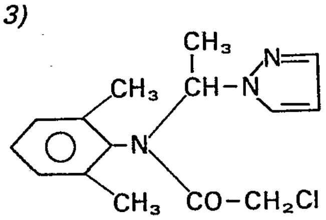

(XII-1)

Zu der in der Stufe 2 erhaltenen Reaktionsmischung werden 170 g (2,5 mol) Pyrazol gegeben, wobei die Temperatur unter 0°C gehalten wird. Durch Zugabe von ca. 300 ml Toluol bleibt die Reaktionsmischung gut rührbar. Man lässt auf Raumtemperatur erwärmen und rührt 3 h nach. Anschliessend wird das Reaktionsgemisch in 1000 ml einer bei 20°C gesättigten, wässerigen Natriumhydrogencarbonatlösung gegeben, die organische Phase wird abgetrennt und filtriert. Das Filtrat wird mehrmals mit gesättigter Natriumhydrogencarbonatlösung gewaschen, über Natriumsulfat getrocknet und im Vakuum eingeengt. Das zurückbleibende Öl wird aus 300 ml Ligroin umkristallisiert. Man erhält 198 g (68% der Theorie, bezogen auf in Stufe 2 eingesetztes N-Ethyliden-2,6-dimethylanilin) 2,6-Dimethyl-N-(1-pyrazol-1-yleth-1-yl)chloracetanilid vom Schmelzpunkt 90°C.

In analoger Weise werden die in der folgenden Tabelle 1 formelmässig aufgeführten Endprodukte der Formel XII hergestellt.

*Tabelle 1*

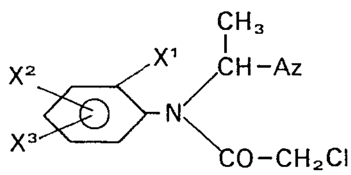

(XII)

| Beispiele Nr. | $X^1$ | $X^2$ | $X^3$ | Az | Schmelzpunkt (°C) |
|---|---|---|---|---|---|
| XII-2 | $CH_3$ | 6-$C_2H_5$ | H | 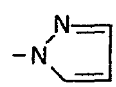 | 84 |
| XII-3 | $C_2H_5$ | 6-$C_2H_5$ | H | 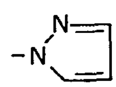 | 80 |
| XII-4 | $CH_3$ | 6-$CH_3$ | H | 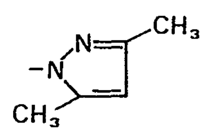 | 86 |
| XII-5 | $CH_3$ | 6-$CH_3$ | H | 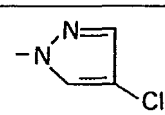 | 92 |
| XII-6 | $CH_3$ | 6-$CH_3$ | H | 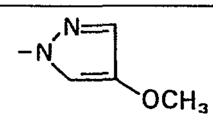 | 85-90 |
| XII-7 | $CH_3$ | 6-$CH_3$ | H | 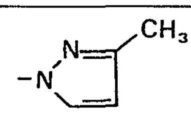 | 79-80 |
| XII-8 | $CH_3$ | 6-$C_2H_5$ | H | 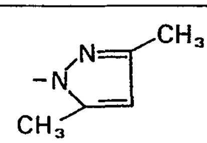 | 122-25 |
| XII-9 | $CH_3$ | 6-$C_2H_5$ | H | 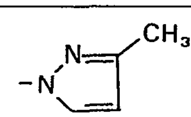 | 102-05 |

Entsprechend Beispiel 1 werden die in der folgenden Tabelle formelmässig aufgeführten Zwischenprodukte der Formel XVII erhalten.

*Tabelle 2*

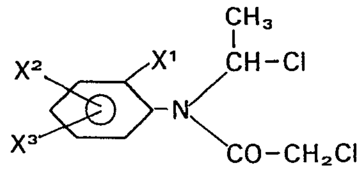

(XVII)

| Beispiele Nr. | $X^1$ | $X^2$ | $X^3$ | Physik. Konstante |
|---|---|---|---|---|
| XVII-2 | $CH_3$ | 6-$C_2H_5$ | H | nicht isoliert |
| XVII-3 | $C_2H_5$ | 6-$C_2H_5$ | H | nicht isoliert |

Entsprechend Beispiel 1 werden die in der folgenden Tabelle formelmässig aufgeführten Zwischenprodukte der Formel XV erhalten.

*Tabelle 3*

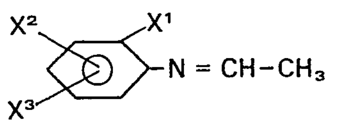

(XV)

| Beispiele Nr. | $X^1$ | $X^2$ | $X^3$ | Siedepunkt (° C/Torr) bzw. Schmelzpunkt |
|---|---|---|---|---|
| XV-2 | $CH_3$ | 6-$C_2H_5$ | H | nicht isoliert |
| XV-3 | $C_2H_5$ | 6-$C_2H_5$ | H | nicht isoliert |

In dem nachfolgenden Beispiel wird die nachstehend angegebene Verbindung als Vergleichssubstanz eingesetzt:

A = 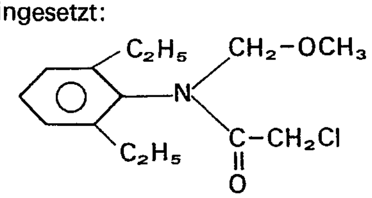

(2,6-Diethyl)-N-methoxymethylchloracetanilid.

*Beispiel A:*

Pre-emergence-*Test*

Lösungsmittel: 5 Gew.-Teile Aceton

Emulgator: 1 Gew.-Teil Alkylarylpolyglykolether

Zur Herstellung einer zweckmässigen Wirkstoffzubereitung vermischt man 1 Gew.-Teil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 h mit der Wirkstoffzubereitung begossen. Dabei hält man die Wassermenge pro Flächeneinheit zweckmässigerweise konstant. Die Wirkstoffkonzentration in der Zubereitung spielt keine Rolle, entscheidend ist nur die Aufwandmenge des Wirkstoffs pro Flächeneinheit. Nach 3 Wochen wird der Schädigungsgrad der Pflanzen bonitiert in %-Schädigung im Vergleich zur Entwicklung der unbehandelten Kontrolle. Es bedeuten:

0% = keine Wirkung (wie unbehandelte Kontrolle)
100% = totale Vernichtung

Der erfindungsgemäss herstellbare Wirkstoff XII-1 zeigt in diesem Test eine bessere selektive Wirksamkeit als die aus dem Stand der Technik bekannte Substanz A.

**Patentanspruch**

Verfahren zur Herstellung von substituierten N-Azolylmethylhalogenacetaniliden der Formel:

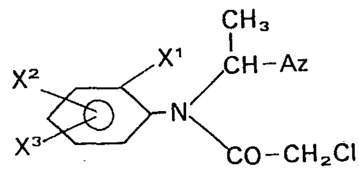

(XII)

in welcher

Az für einen über ein Stickstoffatom gebundenen, gegebenenfalls durch Chlor, Alkyl mit 1 bis 4 Kohlenstoffatomen und/oder Alkoxy mit 1 bis 4 Kohlenstoffatomen, substituierten Pyrazol-1-yl oder 1,2,4-Triazol-1-yl-Rest steht, und

$X^1$, $X^2$ und $X^3$ gleich oder verschieden sind und für Wasserstoff, Alkyl mit 1 bis 4 Kohlenstoffatomen oder für Chlor stehen,

dadurch gekennzeichnet, dass man in einer 1. Stufe Aniline der Formel:

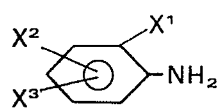

(XIII)

in welcher

$X^1$, $X^2$ und $X^3$ die oben angegebene Bedeutung haben,

mit Acetaldehyd der Formel:

$$H_3C-CH=O \qquad (XIV)$$

gegebenenfalls in Gegenwart eines Verdünnungsmittels und gegebenenfalls in Gegenwart von Dimethylbenzylamin als Katalysator bei Temperaturen zwischen −10 und +120°C umsetzt, dann in einer 2. Stufe die entstehenden Azomethine der Formel:

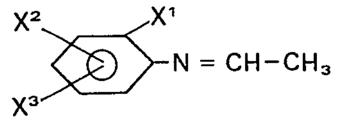

(XV)

in welcher

$X^1$, $X^2$ und $X^3$ die oben angegebene Bedeutung haben,

mit Chloracetylchlorid der Formel:

$$Cl-CO-CH_2-Cl \qquad (XVI)$$

gegebenenfalls in Gegenwart eines Verdünnungsmittels bei Temperaturen zwischen −20 und +50°C umsetzt, und in einer 3. Stufe die entstehenden substituierten N-Halogenmethylhalogenacetanilide der Formel:

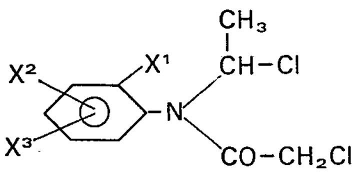

(XVII)

in welcher

$X^1$, $X^2$ und $X^3$ die oben angegebene Bedeutung haben,

ohne vorherige Isolierung direkt mit Azolen der Formel:

H Az (XVIII)

in welcher

Az die oben angegebene Bedeutung hat,

gegebenenfalls in Gegenwart eines Verdünnungsmittels und in Gegenwart eines Säurebindemittels bei Temperaturen zwischen −10 und +80°C umsetzt, wobei die Azole der Formel (XVIII) auch in Form ihrer Alkalimetallsalze eingesetzt werden können.

## Claim

Process for the preparation of substituted N-azolylmethylhalogenoacetanilides of the formula:

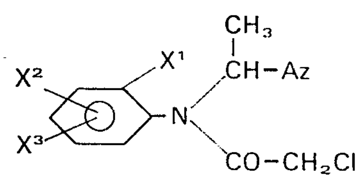

(XII)

in which

Az represents a pyrazol-1-yl or 1,2,4-triazol-1-yl radical which is bonded *via* a nitrogen atom and is optionally substituted by chlorine, alkyl with 1 to 4 carbon atoms and/or alkoxy with 1 to 4 carbon atoms, and

$X^1$, $X^2$ and $X^3$ are identical or different and represent hydrogen, alkyl with 1 to 4 carbon atoms or chlorine,

characterised in that in a 1st stage anilines of the formula:

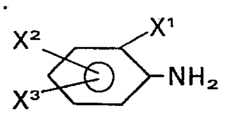

(XIII)

in which

$X^1$, $X^2$ and $X^3$ have the meaning indicated above,

are reacted with acetaldehyde of the formula:

$H_3C-CH = O$ (XIV)

if appropriate in the presence of a diluent and if appropriate in the presence of dimethylbenzylamine as catalyst at temperatures between −10 and +120°C, then in a 2nd stage the resulting azomethines of the formula:

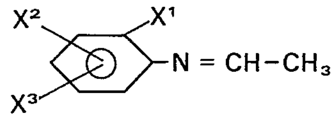

(XV)

in which

$X^1$, $X^2$ and $X^3$ have the meaning indicated above,

are reacted with chloro acetyl chloride of the formula:

$Cl-CO-CH^2Cl$ (XVI)

if appropriate in the presence of a diluent at temperatures between −20 and +50°C, and in a 3rd stage the resulting substituted N-halogenomethylhalogenoacetanilides of the formula:

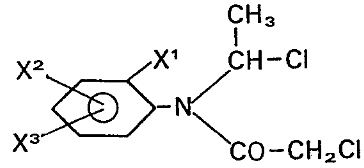

(XVII)

in which

$X^1$, $X^2$ and $X^3$ have the meaning indicated above,

are reacted directly, without prior isolation, with azoles of the formula:

H Az (XVIII)

in which

Az has the meaning indicated above,

if appropriate in the presence of a diluent and in the presence of an acid-binding agent at temperatures between −10 and +80°C, it also being possible to use the azoles of the formula (XVIII) in the form of their alkali metal salts.

## Revendication

Procédé de préparation de N-azolylméthylhalogénacétanilides substitués de formule:

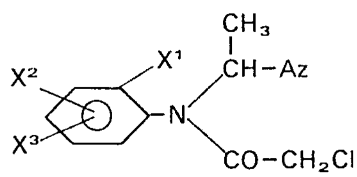

(XII)

dans laquelle

Az représente un radical 1,2,4-triazol-1-yle ou un radical pyrazol-1-yle relié par un atome d'azote et éventuellement substitué par un atome de chlore, par un groupe alkyle contenant 1 à 4 atomes de carbone et/ou par un groupe alcoxy contenant 1 à 4 atomes de carbone, et

$X^1$, $X^2$ et $X^3$ sont identiques ou différents et représentent chacun un atome d'hydrogène, un groupe alkyle contenant 1 à 4 atomes de carbone ou un atome de chlore,

caractérisé en ce que, dans une première étape, on fait réagir des anilines de formule:

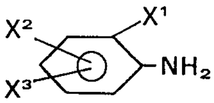

(XIII)

dans laquelle

$X^1$, $X^2$ et $X^3$ ont les significations indiquées ci-dessus,

avec de l'acétaldéhyde de formule:

$$H_3C-CH=O \quad \text{(XIV)}$$

éventuellement en présence d'un diluant et éventuellement en présence de diméthylbenzylamine comme catalyseur à des températures comprises entre −10 et +120°C puis, dans une deuxième étape, on fait réagir les azométhines formées de formule:

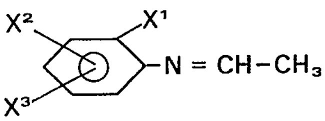

(XV)

dans laquelle

$X^1$, $X^2$ et $X^3$ ont les significations indiquées ci-dessus,

avec du chlorure de chloracétyle de formule:

$$Cl-CO-CH_2Cl \quad \text{(XVI)}$$

éventuellement en présence d'un diluant, à des températures comprises entre −20 et +50°C et, dans une troisième étape, on fait réagir les N-halogénométhylhalogénacétanilides substitués formés de formule:

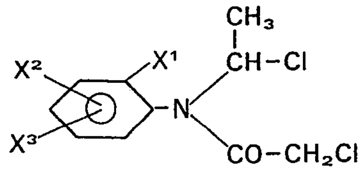

(XVII)

dans laquelle

$X^1$, $X^2$ et $X^3$ ont les significations indiquées ci-dessus,

sans isolation préalable, directement avec des azoles de formule:

$$H\ Az \quad \text{(XVIII)}$$

dans laquelle

Az a la signification indiquée ci-dessus,

éventuellement en présence d'un diluant et en présence d'un agent fixateur d'acide à des températures comprises entre −10 et +80°C, les azoles de formule (XVIII) pouvant également être utilisés sous la forme de leurs sels de métaux alcalins.